# FASCICULE DE BREVET EUROPEEN

(11) **EP 0 761 206 B1**
(45) Date de publication et mention de la délivrance du brevet: **28.01.1998**
(21) Numéro de dépôt: 96401645.5
(22) Date de dépôt: 23.07.1996
(51) Int. Cl.: A61K 7/50, A61K 7/06

(54) **Composition conditionnante et détergente à usage capillaire**
Konditionierende und waschende Haarpflegemittel
Conditioning and washing hair care composition

(30) Priorité: 07.09.1995 FR 9510484
(43) Date de publication de la demande: 12.03.1997
(73) Titulaire: L'OREAL, 75008 Paris (FR)
(72) Inventeur: Cervantes, Frédéric, 75017 Paris (FR); Lopez, Juan, 75020 Paris (FR)
(74) Mandataire: Andral, Christophe André Louis

(56) Documents cités:
- EP-A- 0 152 194
- EP-A- 0 337 354
- EP-A- 0 524 434
- EP-A- 0 538 762
- WO-A-94/07458
- DE-A- 4 229 922
- GB-A- 2 205 747

## Description

La présente invention concerne une composition cosmétique à la fois conditionnante et détergente pour le soin et le lavage simultanés des cheveux.

L'invention concerne aussi l'utilisation de ladite composition dans l'application susmentionnée.

On sait que les cheveux doivent être régulièrement nettoyés de la salissure engendrée par la sécrétion de sébum et l'atmosphère qui les entoure.

Les bases lavantes utilisées à cet effet, et qui sont constituées d'agents tensio-actifs à pouvoir détergent et moussant, le plus souvent des agents tensio-actifs anioniques, débarrassent les cheveux des salissures et du sébum mais laissent ces derniers dans un état difficile à coiffer. Avec ces shampooings traditionnels, les cheveux deviennent secs ou frisottent, manquent d'éclat et de douceur au toucher après leur séchage, sont difficiles à démêler à l'état séché et mouillé. Ils sont parfois aussi chargés d'électricité statique. Ces inconvénients se trouvent encore plus accentués lorsque les cheveux ont été soumis à des traitements chimiques capillaires (coloration, décoloration, permanente,....) ou ont été agressés par la lumière, ou encore lorsqu'ils sont fins.

Pour remédier à ces inconvénients, on a proposé la solution qui consiste à procéder, après le shampooing, et donc dans un deuxième temps, à une application d'agents de conditionnement capillaire, qu'on laisse pauser quelque temps et que l'on rince ensuite à l'eau courante (compositions dites 〈〈 après-shampooings 〉〉). Une autre solution consiste à éviter cette deuxième étape qui prolonge le traitement, ce qui n'est pas souhaitable, en incluant directement un agent de conditionnement capillaire dans le shampooing lui-même (compositions dites 〈〈 shampooings conditionneurs 〉〉.

Des compositions après-shampooings traditionnelles comprennent, à titre d'agents de conditionnement, des alcools gras et des agents tensio-actifs cationiques, et confèrent aux cheveux un toucher doux, une facilité de démêlage et un effet antistatique. Ces après-shampooings destinés à améliorer les propriétés cosmétiques des cheveux, ne présentent, en eux-mêmes, aucune propriété lavante.

Concernant les shampooings conditionneurs précités, les plus courants contiennent, à côté de la base lavante qui est le plus souvent anionique, des agents conditionneurs cationiques.
Toutefois, l'un des problèmes auquel on peut se trouver confronté avec ce genre de composition est celui d'une possible interaction néfaste entre les agents tensio-actifs anioniques, qui sont de bons détergents, et certains agents cationiques de conditionnement, limitant par là l'efficacité de la composition.

Pour éviter ce dernier problème, on a proposé des shampooings conditionneurs contenant des agents tensio-actifs non-ioniques, tels que par exemple les alkylpolyglycosides qui présentent des propriétés moussantes et détergentes intéressantes, en association avec des polymères cationiques et/ou des silicones. De telles compositions sont décrites dans les demandes de brevets EP-0 337 354 et EP-0 398 177. Cependant, ces shampooings ne sont pas encore totalement satisfaisants et conditionnent moins bien les cheveux, et notamment en termes de douceur sur cheveux mouillés, pendant et après l'application, que les compositions après-shampooings décrites ci-avant.

Il existe donc encore un besoin quant à pouvoir disposer d'une composition conditionnante pour les cheveux, qui puisse également les laver dans une même étape et apporter des propriétés conditionnantes au moins égales, si ce n'est supérieures, à celles que présentent les après-shampooings classiques, et qui de plus présentent un caractère de stabilité satisfaisant.

La présente invention vise à satisfaire le besoin ci-dessus en proposant des compositions conditionnantes et détergentes, suffisamment moussantes, et stables, qui présentent des propriétés de conditionnement, et notamment de douceur des cheveux mouillés, égales ou supérieures à celles des compositions après-shampooings classiques à base d'alcools gras et d'agents tensio-actifs cationiques.

Ainsi, après de nombreuses recherches menées sur la question, la Demanderesse a maintenant découvert, de façon totalement inattendue et surprenante, qu'en associant **(A)** un système conditionneur comprenant un alcool gras et un agent tensio-actif cationique particulier, **(B)** une base lavante comprenant un agent tensio-actif non-ionique choisi dans le groupe des alkylpolyglycosides, et **(C)** un système stabilisant spécifique constitué d'un mélange entre du mono-ou-di-stéarate de glycol et un polymère réticulé du chlorure de méthacryloyloxyéthyltriméthylammonium, on pouvait obtenir une composition moussante, stable, qui à la fois conditionne et lave les cheveux, et ceci en apportant des résultats au moins égaux à ceux obtenus par un procédé en deux temps, requérant, dans un premier temps, un lavage avec un shampooing traditionnel, et dans un deuxième temps, l'application d'un après-shampooing comprenant un alcool gras et un agent de conditionnement de type tensio-actif cationique.

Cette découverte est à la base de la présente invention.

La présente invention a ainsi pour objet une nouvelle composition conditionnante et détergente pour les cheveux, qui est caractérisée par le fait qu'elle comprend, en milieu aqueux :
**(A)** un système conditionneur constitué essentiellement de (i) au moins un alcool gras en C₁₄-C₂₂ et (ii) au moins un agent tensio-actif cationique choisi parmi les sels d'ammonium quaternaire, et éventuellement (iii) au moins une silicone cationique,
**(B)** une base lavante constituée essentiellement de (i) au moins un agent tensio-actif non-ionique de type alkylpolyglycoside, et éventuellement (ii) au moins un agent tensio-actif amphotère ou zwittérionique, l'agent tensio-actif amphotère étant plus particulièrement choisi dans le groupe des bétaines,
**(C)** un système stabilisant comprenant (i) au moins un mono-ou-di-stéarate de glycol, et (ii) au moins un polymère réticulé du chlorure de méthacryloyloxyéthyltriméthylammonium.

Une description détaillée de la présente invention va maintenant être donnée.

### A- Système conditionneur

Le ou les alcools gras en C₁₄-C₂₂ qui peuvent être utilisés sont choisis de préférence parmi l'alcool cétylique, l'alcool stéarylique, ou l'alcool cétylstéarylique. Un alcool gras particulièrement préféré selon l'invention est l'alcool cétylstéarylique 50/50 (% calculé en poids).
Le ou les agents tensio-actifs cationiques sont, selon l'invention, des sels d'ammonium quaternaire parmi lesquels on peut citer :
- ceux qui présentent la formule générale (I) suivante : 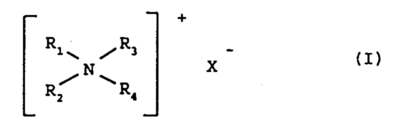 dans laquelle les radicaux R₁ à R₄, qui peuvent être identiques ou différents, représentent un radical aliphatique comportant de 1 à environ 22 atomes de carbone, ou un radical aromatique, alcoxy, polyoxyalkylène, alkylamide, hydroxyalkyle, aryle ou alkylaryle comportant environ de 12 à environ 22 atomes de carbone; X est un anion choisi dans le groupe des halogénures, phosphates, acétates, lactates, alkylsulfates, alkyl-ou-alkylarylsulfonates,
- les sels d'ammonium quaternaire de l'imidazolinium, comme par exemple celui de formule (II) suivante : 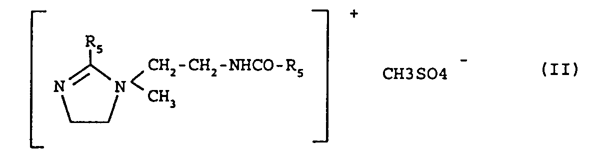 dans laquelle R₅ représente un mélange de radicaux alcényle et/ou alkyle comportant de 13 à 21 atomes de carbone et dérivés des acides gras du suif, tel que le produit vendu sous la marque 〈〈REWOQUAT W 7500〉〉 par la société REWO,
- les sels de diammonium quaternaire de formule (III) : 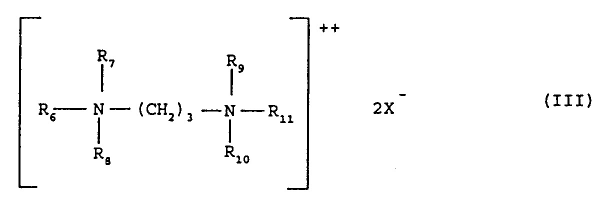 dans laquelle R₆ désigne un radical aliphatique comportant environ de 16 à 22 atomes de carbone, R₇, R₈, R₉, R₁₀, et R₁₁ sont choisis parmi l'hydrogène ou un radical alkyle comportant de 1 à 4 atomes de carbone, et X est un anion choisi dans le groupe des halogénures, acétates, phosphates, et sulfates. De tels sels de diammonium quaternaire comprennent notamment le dichlorure de propanesuif diammonium.

Parmi les sels d'ammonium quaternaire de formule (I) on préfère, d'une part, les chlorures de tétraalkylammonium comme par exemple les chlorures de dialkyldiméthylammonium ou d'alkyltriméthylammonium, dans lesquels le radical alkyl comporte environ de 12 à environ 22 atomes de carbone, en particulier les chlorures de béhényltriméthylammonium, de distéaryldiméthylammonium ou de cétyltriméthylammonium, ou encore, d'autre part, le chorure de stéaramidopropyldiméthyl (myristyl acetate) ammonium vendu sous la marque 〈〈CERAPHYL 70〉〉 par la société VAN DYK.

Selon l'invention, le chlorure de béhényltriméthylammonium est le sel d'ammonium quaternaire le plus particulièrement préféré.

Selon l'invention, le système conditionneur peut en outre éventuellement contenir une silicone cationique.

De telles silicones cationiques comprennent notamment le polymère de formule (IV): 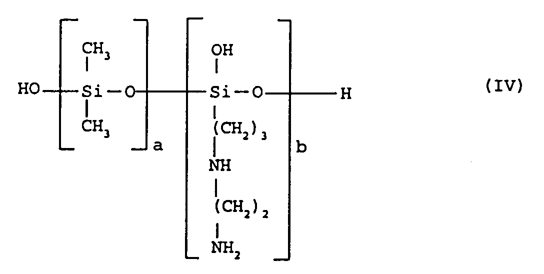 dans laquelle a et b sont des nombres entiers dépendant du poids moléculaire, le poids moléculaire moyen étant approximativement compris entre 5000 et 10000.
Ce polymère est connu sous la dénomination 〈〈 Amodiméthicone 〉〉 dans le dictionnaire C.T.F.A.
Un produit comprenant ce type de polymère, et qui est ici plus particulièrement préféré, est celui vendu sous la dénomination commerciale 〈〈 Emulsion cationique DC 939 〉〉 (ou DC 939) par la société Dow Corning, qui est une asssociation d'Amodiméthicone, de chlorure d'hexadécyltriméthylammonium et d'alcool tridécylique polyoxyéthyléné, en émulsion aqueuse à 36% d'Amodiméthicone.

D'autres silicones cationiques qui peuvent être utilisées selon la présente invention sont des polymères de formule (V) :

(R₁₂)_{c} G_{3-c}-Si-(-OSi G₂)ₙ-[OSi G_{d} (R₁₂)_{2-d}]ₘ-O-Si G_{3-c} (R₁₂)_{c} (V)

dans laquelle
- G est choisi dans le groupe formé par H, OH, alkyle en C₁₋₈, et phényle, et désigne de préférence méthyle,
- a désigne 0 ou un nombre entier allant de 1 à 3, et de préférence est égal à 0,
- b désigne 0 ou 1, et de préférence est égal à 1,
- la somme (n+m) représente un nombre allant de 0 à 2000 et de préférence allant de 50 à 150, n pouvant désigner un nombre allant de 0 à 1999 et de préférence allant de 49 à 149 et m pouvant désigner un nombre allant de 1 à 2000 et de préférence allant de 1 à 10,
- R₁₂ est un radical monovalent de formule C_{q} H_{2q} L dans laquelle q = 2 à 8, L étant choisi parmi les groupements :
   - N(R₁₃)CH₂ - CH₂ - N(R₁₃)₂
   - N(R₁₃)₂
   - N^{⊕}(R₁₃)₃A⁻
   - N^{⊕}(R₁₃)CH₂ - CH₂ - N^{⊕}( R₁₃)H₂ A⁻
   dans lesquels R₁₃ est choisi dans le groupe formé par H, phényle, benzyle, un radical hydrocarboné saturé, de préférence un radical alkyle comportant de 1 à 20 atomes de carbone, et A⁻ désigne un ion halogénure.
Ces composés sont décrits plus en détail dans la demande de brevet européen EP-A-095 238.
Un polymère de formule (V) plus particulièrement préféré est connu dans le dictionnaire C.T.F.A. sous la dénomination 〈〈 Triméthylsilylamodiméthicone 〉〉 de formule (VI) : 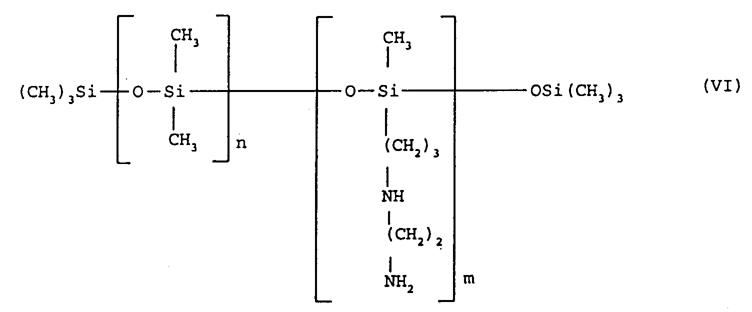 dans laquelle n et m ont les mêmes significations que ci-dessus.
Un produit comprenant ce type de polymère plus particulièrement préféré est celui vendu par la société Dow Corning sous la dénomination 〈〈 Dow Corning Q2 7224 〉〉.

D'autres silicones cationiques pouvant encore être utilisées dans la composition selon la présente invention sont des polymères de formule (VII) : 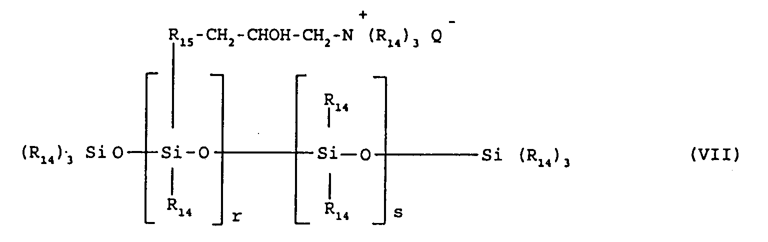 dans laquelle,
- R₁₄ désigne un radical hydrocarboné comportant de 1 à 18 atomes de carbone, en particulier un radical alkyle ou alkényle et de préférence méthyle,
- R₁₅ désigne un radical hydrocarboné, de préférence un radical alkylène comportant de 1 à 18 atomes de carbone ou un radical alkylèneoxy comportant de 1 à 18 atomes de carbone et de préférence de 1 à 8 atomes de carbone,
- Q⁻ désigne un ion halogénure et de préférence un ion chlorure,
- r représente une valeur statistique moyenne allant de 2 à 20 et de préférence de 2 à 8,
- s représente une valeur statistique moyenne allant de 20 à 200 et de préférence de 20 à 50.
Ces composés sont décrits plus en détail dans le brevet US- 4 185 087.
Un polymère de ce type plus particulièrement préféré est celui vendu par la société Union Carbide sous la dénomination 〈〈 Ucar Silicone Ale 56 〉〉.

Dans la composition conditionnante et détergente selon la présente invention, le système conditionneur représente généralement (en concentrations pondérales ramenées à l'ensemble de la composition) : (i) alcool(s) gras C₁₄-C₂₂ : entre environ 1 et 10%, de préférence entre environ 1 et 8%; (ii) agent(s) tensio-actif(s) cationique(s) de type sel d'ammonium quaternaire : entre environ 0,3 et 10%, de préférence entre environ 0,5 et 8%; (iii) silicone(s) cationique(s) : 0 et environ 10%.

### B- Base lavante

Le ou les agents tensio-actifs non-ioniques de type alkylpolyglycoside, utilisés dans le cadre de la présente invention, sont des produits bien connus en soi, et ils peuvent être plus particulièrement représentés par la formule générale (VIII) suivante :

R₁₆-O-(R₁₇O)ₜ-(G)ᵥ (VIII)

dans laquelle R₁₆ représente un radical alkyle et/ou alcényle linéaire ou ramifié comportant environ de 8 à 24 atomes de carbone, un radical alkylphényle dont le radical alkyle linéaire ou ramifié comporte environ de 8 à 24 atomes de carbone, R₁₇ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15.

Des alkylpolyglycosides préférés selon la présente invention sont des composés de formule (VIII) dans laquelle R₁₆ désigne plus particulièrement un radical alkyle et/ou alcényle linéaire ou ramifié comportant de 9 à 14 atomes de carbone, t désigne une valeur allant de 0 à 3 et plus particulièrement encore égale à 0, G désigne le glucose, le fructose ou le galactose. Le degré de polymérisation (S) du saccharide, i.e. la valeur de v dans la formule (VIII), peut aller de 1 à 15. Selon l'invention, on préfère les sucres réduits contenant 80%, ou plus, de sucres dont le degré de polymérisation (S) prend une valeur allant de 1 à 4.

Des composés de formule (VIII) sont notamment représentés par les produits vendus par la société HENKEL sous la dénomination APG, tels que les produits APG 300, APG 350, APG 500, APG 550, APG 625, APG base 10-12; les produits vendus par la société SEPPIC sous les dénominations TRITON CG 110 (ou ORAMIX CG 110) et TRITON CG 312 (ou ORAMIX NS 10); ceux vendus par la société B.A.S.F. sous la dénomination LUTENSOL GD 70.

Il est également possible selon l'invention d'associer au tensio-actif non-ionique de type alkylpolyglycoside, un agent tensio-actif de type amphotère ou zwittérionique.

Les agents tensioactifs amphotères ou zwittérioniques, dont la nature ne revêt pas dans le cadre de la présente invention de caractère critique, peuvent être notamment (liste non limitative):
- des dérivés d'amines secondaires ou tertiaires aliphatiques, dans lesquels le radical aliphatique est une chaîne linéaire ou ramifiée comportant de 8 à 18 atomes de carbone et contenant au moins un groupe anionique hydrosolubilisant (par exemple carboxylate, sulfonate, sulfate, phosphate ou phosphonate),
- des alkyl (C₈-C₂₀) bétaïnes, des sulfobétaïnes, des alkyl (C₈-C₂₀) amidoalkyl (C₁-C₆) bétaïnes ou des alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates.

Selon la présente invention, on préfère plus particulièrement utiliser les agents tensio-actifs amphotères appartenant au groupe des bétaines.

Dans la composition conditionnante et détergente selon la présente invention, la base lavante représente généralement (en concentrations pondérales ramenées à l'ensemble de la composition) : (i) agent(s) tensio-actif(s) non-ionique(s) de type alkylpolyglycoside : entre environ 5 et 30%, de préférence entre environ 8 et 25%; (ii) agent(s) tensio-actif(s) amphotère(s) ou zwittérionique(s) : 0 et environ 10%.

### C- Système stabilisant

Selon une caractéristique essentielle de la présente invention, le système stabilisant comprend un mono-ou-di-stéarate de glycol (les stéarates du commerce renferment 70% en poids de chaînes en C₁₈ et 30% en poids de chaînes en C₁₆), associé à un polymère réticulé du chlorure de méthacryloyloxyéthyltriméthylammonium. Des polymères de ce type sont notamment vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyltriméthylammonium).
Un polymère plus particulièrement préféré selon la présente invention est le copolymère du chlorure de méthacryloyloxyéthyltriméthylammonium et de l'acrylamide 42/58 (% exprimé en poids).

Dans la composition conditionnante et détergente selon la présente invention, le système stabilisant représente généralement (en concentrations pondérales ramenées à l'ensemble de la composition) : (i) mono-ou-di-stéarate de glycol : entre environ 0,1 et 6%, et de préférence entre environ 0,2 et 4%; (ii) polymère réticulé du chlorure de méthacryloyloxyéthyltriméthylammonium : entre environ 0,2 et 3%, et de préférence entre environ 0,4 et 2%.

La composition conditionnante et détergente selon la présente invention peut encore contenir, en plus des trois systèmes (A), (B) et (C) décrits ci-avant qui le caractérisent, d'autres ingrédients ou actifs bien connus dans le domaine des shampooings ou après-shampooings pourvu bien entendu que leur introduction dans la composition n'en altère ni la stabilité ni les propriétés visées par la présente invention. Parmi ces composés, on peut citer à titre d'exemple, des alcools gras polyoxyéthylénés, des esters gras, des vitamines, des solvants, des agents séquestrants, des épaississants, des adoucissants, des agents modificateur de mousse, des agents nacrants, des agents hydratants, des agents antipelliculaires ou antiséborrhéiques, des filtres solaires, des colorants, des parfums, des conservateurs et autres.

La composition selon l'invention peut se présenter sous forme de liquide plus ou moins épaissi, de crème ou de gel.

Des exemples concrets illustrant l'invention vont maintenant être donnés.

### EXEMPLE 1:

On a préparé une composition conditionnante et détergente selon l'invention de composition suivante (MA signifie Matière Active):

| | |
|---|---|
| - Alkyl (C9/C11) polyglycoside (1,4) en solution aqueuse à 40% | 14 g MA |
| - Cocoyl amidopropyl diméthyl hydroxypropyl sulfobétaine en solution aqueuse à 50% | 2,5 g MA |
| - Alcool cétylstéarylique (C16/C18) 50/50 (% en poids) | 5 g |
| - Copolymère de chlorure de méthacryloyloxyéthyltriméthylammonium et d' acrylamide 42/58 (% exprimé en poids), vendu par la société ALLIED COLLOIDS en dispersion à 50% dans de l'huile minérale, sous la dénomination SALCARE SC 92 | 0,5 g MA |
| - Di-stéarate de glycol (C16/C18) 30/70 (% en poids) | 2 g MA |
| - Chlorure de béhényltriméthylammonium à 80% en poids dans un mélange eau/isopropanol 15/85 (% exprimé en poids) | 2,4 g MA |
| - Silicone cationique : 〈〈 Emulsion Cationique DC 939 〉〉 de la société Dow Corning | 0,88 g MA |
| - Conservateurs, parfums, | qs |
| - Eau déminéralisée qsp | 100 g |

Cette composition s'est révélée stable deux mois à la température de 45°C.

Des cheveux ont été lavés à l'aide de cette composition. On constate qu'ils sont très doux pendant cette application. Ils ont ensuite été rincés à l'eau courante. Avant le séchage, on constate qu'ils sont aussi très doux à l'état mouillé et se démêlent bien.

### EXEMPLES COMPARATIFS 2 et 3 :

On a reproduit la composition de l'exemple 1 mais sans introduire de di-stéarate de glycol.
La composition résultante (exemple 2) s'est déstabilisée dès 3 jours à la température de 45°C.

On a reproduit la composition de l'exemple 1 mais sans introduire de copolymère de chlorure de méthacryloyloxyéthyltriméthylammonium et d' acrylamide 42/58 (SALCARE SC92).
La composition résultante (exemple 3) s'est déstabilisée en 13 jours à la température de 45°C.

## Revendications

1. Composition conditionnante et détergente à usage capillaire, caractérisée par le fait qu'elle comprend, en milieu aqueux :
**(A)** un système conditionneur constitué essentiellement de (i) au moins un alcool gras en C₁₄-C₂₂ et (ii) au moins un agent tensio-actif cationique choisi parmi les sels d'ammonium quaternaire, et éventuellement (iii) au moins une silicone cationique,
**(B)** une base lavante constituée essentiellement de (i) au moins un agent tensio-actif non-ionique de type alkylpolyglycoside, et éventuellement (ii) au moins un agent tensio-actif amphotère ou zwittérionique,
**(C)** un système stabilisant comprenant (i) au moins un mono-ou-di-stéarate de glycol, et (ii) au moins un polymère réticulé du chlorure de méthacryloyloxyéthyltriméthylammonium.

2. Composition selon la revendication 1, caractérisée par le fait que ledit système conditionneur **(A)** comprend une silicone cationique.

3. Composition selon les revendications 1 ou 2, caractérisée par le fait que ladite base lavante **(B)** comprend un agent tensio-actif amphotère ou zwittérionique.

4. Composition selon l'une quelconque des revendications précédentes, caractérisée par le fait que ledit alcool gras en C₁₄-C₂₂ est choisi parmi l'alcool cétylique, l'alcool stéarylique, l'alcool cétylstéarylique, et leurs mélanges.

5. Composition selon l'une quelconque des revendications précédentes, caractérisée par le fait que ledit agent tensio-actif cationique de type sel d'ammonium quaternaire est choisi parmi ceux qui présentent la formule générale (I) suivante : 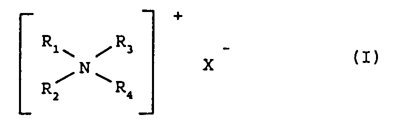 dans laquelle R₁ à R₄, identiques ou différents, représentent un radical aliphatique comportant de 1 à 22 atomes de carbone, ou un radical aromatique, alcoxy, polyoxyalkylène, alkylamide, hydroxyalkyle, aryle ou alkylaryle comportant de 12 à 22 atomes de carbone; X est un anion choisi dans le groupe des halogénures, phosphates, acétates, lactates ou alkylsulfates,
ou les sels d'ammonium quaternaire de l'imidazolinium, de formule (II) suivante : 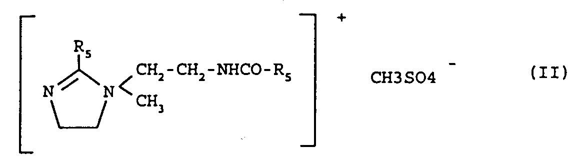 dans laquelle R₅ représente un mélange de radicaux alcényle et/ou alkyle comportant de 13 à 21 atomes de carbone et dérivés des acides gras du suif,
ou les sels de diammonium quaternaire de formule (III) : 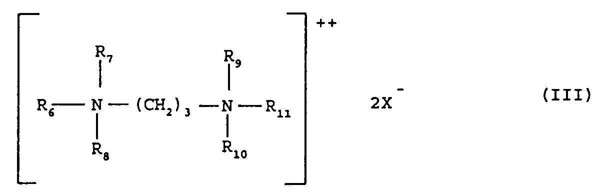 dans laquelle R₆ désigne un radical aliphatique comportant de 16 à 22 atomes de carbone, R₇, R₈, R₉, R₁₀, et R₁₁ sont choisis parmi l'hydrogène ou un radical alkyle comportant de 1 à 4 atomes de carbone, et X est un anion choisi dans le groupe des halogénures, acétates, phosphates, et sulfates.

6. Composition selon la revendication 5, caractérisée par le fait que ledit sel d'ammonium quaternaire de formule (I) est un chlorure de dialkyldiméthylammonium ou d'alkyltriméthylammonium, dans lesquels le radical alkyle comporte de 12 à 22 atomes de carbone.

7. Composition selon la revendication 6, caractérisée par le fait que ledit chlorure de dialkyldiméthylammonium ou d'alkyltriméthylammonium est un chlorure de distéaryldiméthylammonium, de cétyltriméthylammonium, ou de béhényltriméthylammonium.

8. Composition selon la revendication 5, caractérisée par le fait que ledit sel d'ammonium quaternaire est le chlorure de stéaramidopropyldiméthyl(myristyl acetate) ammonium.

9. Composition selon la revendication 7, caractérisée par le fait que ledit chlorure d'alkyltriméthylammonium est le chlorure de béhényltriméthylammonium.

10. Composition selon l'une quelconque des revendications précédentes, caractérisée par le fait que ladite silicone cationique est une Amodiméthicone de formule (IV) : 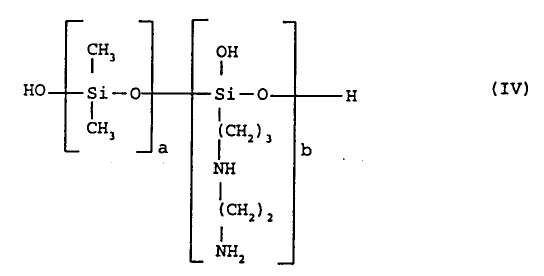 dans laquelle a et b sont des nombres entiers dépendant du poids moléculaire, le poids moléculaire moyen étant approximativement compris entre 5000 et 10000.

11. Composition selon la revendication 10, caractérisée par le fait que l'Amodiméthicone est associé au chlorure d'hexadécyltriméthylammonium et à l'alcool tridécylique polyoxyéthyléné.

12. Composition selon l'une quelconque des revendications précédentes, caractérisée par le fait que ledit agent tensio-actif non-ionique de type alkylpolyglycoside est un composé de formule (VIII) :
R₁₆-O-(R₁₇O)ₜ-(G)ᵥ (VIII)
dans laquelle R₁₆ représente un radical alkyle et/ou alcényle linéaire ou ramifié comportant de 8 à 24 atomes de carbone, un radical alkylphényle dont le radical alkyle linéaire ou ramifié comporte de 8 à 24 atomes de carbone, R₁₇ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15.

13. Composition selon la revendication 12, caractérisée par le fait que dans la formule (VIII), R₁₆ désigne un radical alkyle et/ou alcényle linéaire ou ramifié comportant de 9 à 14 atomes de carbone, t prend la valeur 0, G désigne le glucose, v prend une valeur de 1 à 4.

14. Composition selon l'une quelconque des revendications précédentes, caractérisée par le fait que ledit agent tensio-actif amphotère ou zwittérionique est choisi dans le groupe des bétaines et notamment parmi les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

15. Composition selon l'une quelconque des revendications précédentes, caractérisée par le fait que le polymère réticulé du chlorure de méthacryloyloxyéthyltriméthylammonium est un copolymère réticulé du chlorure de méthacryloyloxyéthyltriméthylammonium et de l'acrylamide.

16. Composition selon l'une quelconque des revendications précédentes, caractérisée par le fait que ledit alcool gras en C₁₄-C₂₂ est présent dans des concentrations pondérales comprises entre 1 et 10% par rapport au poids total de la composition.

17. Composition selon l'une quelconque des revendications précédentes, caractérisée par le fait que ledit agent tensio-actif cationique de type sel d'ammonium quaternaire est présent dans des concentrations pondérales comprises entre 0,3 et 10% par rapport au poids total de la composition.

18. Composition selon l'une quelconque des revendications pécédentes, caractérisée par le fait que ladite silicone cationique est présente dans des concentrations pondérales comprises entre 0 et 10% par rapport au poids total de la composition.

19. Composition selon l'une quelconque des revendications précédentes, caractérisée par le fait que ledit agent tensio-actif non-ionique de type alkylpolyglycoside est présent dans des concentrations pondérales comprises entre 5 et 30% par rapport au poids total de la composition.

20. Composition selon l'une quelconque des revendications précédentes, caractérisée par le fait que ledit agent tensio-actif amphotère ou zwittérionique est présent dans des concentrations pondérales comprises entre 0 et 10% par rapport au poids total de la composition.

21. Composition selon l'une quelconque des revendications précédentes, caractérisée par le fait que ledit mono-ou di-stéarate de glycol est présent dans des concentrations pondérales comprises entre 0,1 et 6% par rapport au poids total de la composition.

22. Composition selon l'une quelconque des revendications précédentes, caractérisée par le fait que ledit polymère réticulé du chlorure de méthacryloyloxyéthyltriméthylammonium est présent dans des concentrations pondérales comprises entre 0,2 et 3% par rapport au poids total de la composition.

23. Composition selon l'une quelconque des revendications précédentes, caractérisée par le fait qu'elle se présente sous la forme d'un liquide plus ou moins épaissi, d'une crème ou d'un gel.

24. Utilisation de la composition telle que définie à l'une quelconque des revendications précédentes pour le soin et le lavage simultanés des cheveux.

## Claims

1. Conditioning and detergent composition for use on hair, characterized in that it comprises, in an aqueous medium:
(A) a conditioner system consisting essentially of (i) at least one C₁₄-C₂₂ fatty alcohol and (ii) at least one cationic surfactant chosen from quaternary ammonium salts, and optionally (iii) at least one cationic silicone,
(B) a washing base consisting essentially of (i) at least one nonionic surfactant of the alkylpolyglycoside type, and optionally (ii) at least one amphoteric or zwitterionic surfactant, and
(C) a stabilizer system comprising (i) at least one glycol mono- or distearate and (ii) at least one crosslinked polymer of methacryloyloxyethyltrimethylammonium chloride.

2. Composition according to Claim 1, characterized in that the said conditioner system (A) comprises a cationic silicone.

3. Composition according to Claims 1 or 2, characterized in that the said washing base (B) comprises an amphoteric or zwitterionic surfactant.

4. Composition according to any one of the preceding claims, characterized in that the said C₁₄-C₂₂ fatty alcohol is chosen from cetyl alcohol, stearyl alcohol, cetyl stearyl alcohol and mixtures thereof.

5. Composition according to any one of the preceding claims, characterized in that the said cationic surfactant of the quaternary ammonium salt type is chosen from those having the following general formula (I): 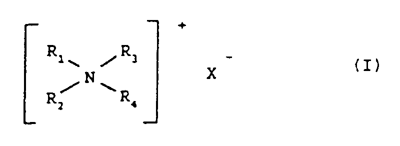 in which R₁ to R₄, which can be identical or different, are an aliphatic radical containing 1 to 22 carbon atoms, or an aromatic, alkoxy, polyoxyalxylene, alkylamide, hydroxyalkyl, aryl or alkylaryl radical containing 12 to 22 carbon atoms; X is an anion chosen from the group consisting of halides, phosphates, acetates, lactates or alkyl sulphates,
or quaternary ammonium salts of the imidazolinium type, of the following formula (II): 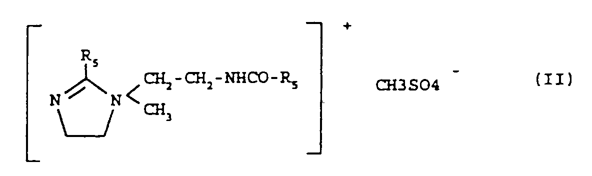 in which R₅ is a mixture of alkenyl and/or alkyl radicals containing 13 to 21 carbon atoms and derivatives of tallow fatty acids,
or the quaternary diammonium salts of formula (III): 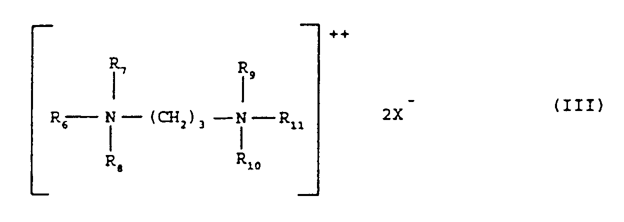 in which R₆ is an aliphatic radical containing 16 to 22 carbon atoms, R₇, R₈, R₉, R₁₀ and R₁₁ are chosen from hydrogen or an alkyl radical containing 1 to 4 carbon atoms, and X is an anion chosen from the group consisting of halides, acetates, phosphates and sulphates.

6. Composition according to Claim 5, characterized in that the said quaternary ammonium salt of formula (I) is a dialkyldimethylammonium or alkyltrimethylammonium chloride in which the alkyl radical contains 12 to 22 carbon atoms.

7. Composition according to Claim 6, characterized in that the said dialkyldimethylammonium or alkyltrimethylammonium chloride is distearyldimethylammonium chloride, cetyltrimethylammonium chloride or behenyltrimethylammonium chloride.

8. Composition according to Claim 5, characterized in that the said quaternary ammonium salt is stearamidopropyldimethyl(myristyl acetate)ammonium chloride.

9. Composition according to Claim 7, characterized in that the said alkyltrimethylammonium chloride is behenyltrimethylammonium chloride.

10. Composition according to any one of the preceding claims, characterized in that the said cationic silicone is an Amodimethicone of formula (IV): 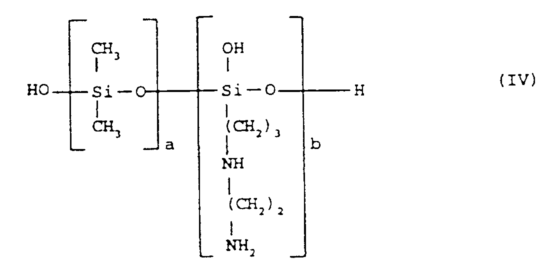 in which a and b are integers depending on the molecular weight, the average molecular weight being approximately between 5000 and 10,000.

11. Composition according to Claim 10, characterized in that Amodimethicone is combined with hexadecyltrimethylammonium chloride and with polyethoxylated tridecyl alcohol.

12. Composition according to any one of the preceding claims, characterized in that the said nonionic surfactant of the alkylpolyglycoside type is a compound of formula (VIII):
R₁₆-O-(R₁₇O)ₜ-(G)ᵥ (VIII)
in which R₁₆ is a linear or branched alkyl and/or alkenyl radical containing 8 to 24 carbon atoms, an alkylphenyl radical whose linear or branched alkyl radical contains 8 to 24 carbon atoms, R₁₇ is an alkylene radical containing 2 to 4 carbon atoms, G is a reduced sugar containing 5 to 6 carbon atoms, t is a value ranging from 0 to 10 and v is a value ranging from 1 to 15.

13. Composition according to Claim 12, characterized in that in the formula (VIII) R₁₆ is a linear or branched alkyl and/or alkenyl radical containing 9 to 14 carbon atoms, t adopts the value 0, G is glucose and v has a value from 1 to 4.

14. Composition according to any one of the preceding claims, characterized in that the said amphoteric or zwitterionic surfactant is chosen from the group of the betaines and, in particular, from (C₈-C₂₀ alkyl)betaines, sulphobetaines, (C₈-C₂₀ alkyl)amido(C₁-C₆ alkyl)betaines or (C₈-C₂₀ alkyl) amido (C₁-C₆ alkyl)sulphobetaines.

15. Composition according to any one of the preceding claims, characterized in that the crosslinked polymer of methacryloyloxyethyltrimethylammonium chloride is a crosslinked copolymer of methacryloyloxyethyltrimethylammonium chloride and acrylamide.

16. Composition according to any one of the preceding claims, characterized in that the said C₁₄-C₂₂ fatty alcohol is present in concentrations by weight of between 1 and 10 % relative to the total weight of the composition.

17. Composition according to any one of the preceding claims, characterized in that the said cationic surfactant of quaternary ammonium salt type is present in concentrations by weight of between 0.3 and 10 % relative to the total weight of the composition.

18. Composition according to any one of the preceding claims, characterized in that the said cationic silicone is present in concentrations by weight of between 0 and 10 % relative to the total weight of the composition.

19. Composition according to any one of the preceding claims, characterized in that the said nonionic surfactant of the alkylpolyglycoside type is present in concentrations by weight of between 5 and 30 % relative to the total weight of the composition.

20. Composition according to any one of the preceding claims, characterized in that the said amphoteric or zwitterionic surfactant is present in concentrations by weight of between 0 and 10 % relative to the total weight of the composition.

21. Composition according to any one of the preceding claims, characterized in that the said glycol mono- or distearate is present in concentrations by weight of between 0.1 and 6 % relative to the total weight of the composition.

22. Composition according to any one of the preceding claims, characterized in that the said crosslinked polymer of methacryloyloxyethyltrimethylammonium chloride is present in concentrations by weight of between 0.2 and 3 % relative to the total weight of the composition.

23. Composition according to any one of the preceding claims, characterized in that it is in the form of a more or less thick liquid or a cream or gel.

24. Use of the composition as defined in any one of the preceding claims for the simultaneous care and cleaning of the hair.

## Patentansprüche

1. Konditionierende und reinigende Zusammensetzung zur Verwendung bei der Haarpflege, dadurch gekennzeichnet, daß sie in einem wässerigen Medium umfaßt:
(A) ein Konditioniersystem, das im wesentlichen aus (i) mindestens einem C₁₄₋₂₂-Fettalkohl, (ii) mindestens einem kationischen Tensid, das aus den quaternären Ammoniumsalzen ausgewählt ist, und gegebenenfalls (iii) mindestens einem kationischen Silicon besteht,
(B) einen Waschrohstoff, der im wesentlichen aus (i) mindestens einem nichtionischen Tensid vom Alkylpolyglykosidtyp und gegebenenfalls (ii) mindestens einem amphoteren oder zwitterionischen Tensid besteht
und
(C) ein stabilisierendes System, das (i) mindestens ein Glykolmonostearat oder ein Glykoldistearat und (ii) mindestens ein vernetztes Methacryloyloxyethyltrimethylammoniumchlorid-Polymer umfaßt.

2. Zusammensetzung nach Anspruch 1, dadurch gekennzeichnet, daß das Konditioniersystem (A) ein kationisches Silicon umfaßt.

3. Zusammmensetzung nach Anspruch 1 oder 2, dadurch gekennzeichnet, daß der Waschrohstoff (B) ein amphoteres oder zwitterionisches Tensid umfaßt.

4. Zusammmensetzung nach einem der vorhergehenden Ansprüche, dadurch gekennzeichnet, daß der C₁₄₋₂₂-Fettalkohol unter Cetylalkohol, Stearylalkohol, Cetylstearylalkohol und den Gemischen dieser Alkohole ausgewählt ist.

5. Zusammmensetzung nach einem der vorhergehenden Ansprüche, dadurch gekennzeichnet, daß das kationische Tensid vom Typ eines quaternären Ammoniumsalzes ausgewählt ist unter den Verbindungen der folgenden allgemeinen Formel (I): 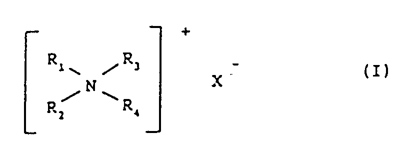 worin bedeuten:
- R₁ bis R₄, die identisch oder voneinander verschieden sind, eine aliphatische Gruppe mit 1 bis 22 Kohlenstoffatomen oder eine aromatische Gruppe, Alkoxy, Polyoxyalkylen, Alkylamid, Hydroxyalkyl, Aryl oder Alkylaryl mit 12 bis 22 Kohlenstoffatomen und
- X ein Anion, das aus der Gruppe der Halogenide, Phosphate, Actetate, Lactate oder Alkylsulfate ausgewählt ist,
oder unter den quaternären Ammoniumsalzen von Imidazolinium der folgenden Formel (II): 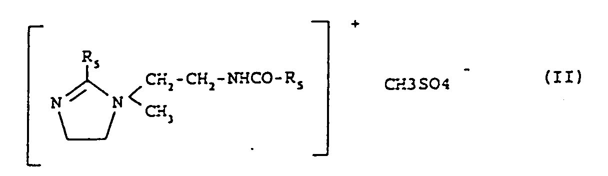 worin R₅ ein Gemisch aus Alkenylgruppen und/oder Alkylgruppen mit 13 bis 21 Kohlenstoffatomen, die von Talgfettsäuren abgeleitet sind, bedeutet,
oder unter den quaternären Diammoniumsalzen der Formel (III): 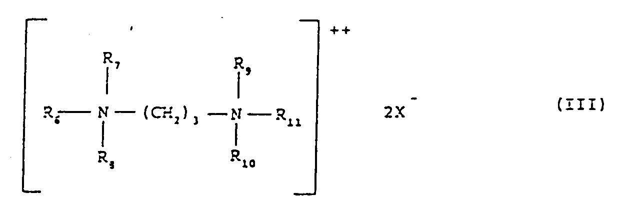 worin:
- R₆ eine aliphatische Gruppe mit 16 bis 22 Kohlenstoffatomen bezeichnet,
- R₇, R₈, R₉, R₁₀ und R₁₁ aus Wasserstoff oder einer Alkylgruppe mit 1 bis 4 Kohlenstoffatomen ausgewählt sind
und
- X ein Anion ist, das aus der Gruppe der Halogenide, Acetate, Phosphate und Sulfate ausgewählt ist.

6. Zusammmensetzung nach Anspruch 5, dadurch gekennzeichnet, daß das quaternäre Ammoniumsalz der Formel (I) ein Dialkyldimethylammoniumchlorid-oder ein Akyltrimethylammoniumchlorid ist, worin die Alkylgruppe 12 bis 22 Kohlenstoffatome aufweist.

7. Zusammmensetzung nach Anspruch 6, dadurch gekennzeichnet, daß das Dialkyldimethylammoniumchlorid oder das Akyltrimethylammoniumchlorid ein Distearyldimethylammoniumchlorid, ein Cetyltrimethylammoniumchlorid oder ein Behenyltrimethylammoniumchlorid ist.

8. Zusammmensetzung nach Anspruch 5, dadurch gekennzeichnet, daß das quaternäre Ammoniumsalz Stearamidopropyldimethyl-(myristylacetat)-ammoniumchlorid ist.

9. Zusammmensetzung nach Anspruch 7, dadurch gekennzeichnet, daß das Alkyltrimethylammoniumchlorid Behenyltrimethylammoniumchlorid ist.

10. Zusammmensetzung nach einem der vorhergehenden Ansprüche, dadurch gekennzeichnet, daß das kationische Silicon ein Amodimeticon der Formel (IV) ist: 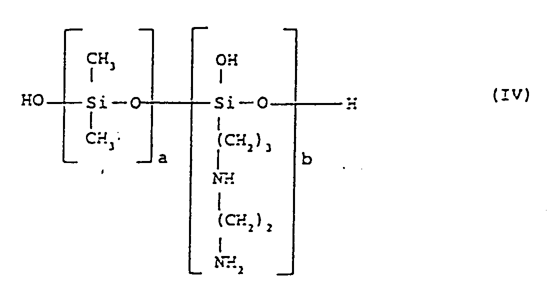 worin a und b ganze Zahlen sind, die vom Molekülgewicht abhängen, wobei das mittlere Molekülgewicht ungefähr im Bereich von 5000 bis 10000 liegt.

11. Zusammmensetzung nach Anspruch 10, dadurch gekennzeichnet, daß das Amodimeticon mit Hexadecyltrimethylammoniumchlorid und polyethoxyliertem Tridecylalkohol kombiniert ist.

12. Zusammmensetzung nach einem der vorhergehenden Ansprüche, dadurch gekennzeichnet, daß das nichtionische Tensid vom Alkylpolyglykosidtyp eine Verbindung der Formel (VIII) ist:
R₁₆-O-(R₁₇O)ₜ-(G)ᵥ (VIII)
worin bedeuten:
- R₁₆ eine geradkettige oder verzweigte Alkylgruppe und/oder Alkenylgruppe mit 8 bis 24 Kohlenstoffatomen oder eine Alkylphenylgruppe, deren geradkettige oder verzweigte Alkylgruppe 8 bis 24 Kohlenstoffatome aufweist,
- R₁₇ eine Alkylengruppe mit 2 bis 4 Kohlenstoffatomen,
- G einen reduzierten Zucker mit 5 bis 6 Kohlenstoffatomen,
- t einen Wert von Null bis 10
und
- v einen Wert von 1 bis 15.

13. Zusammmensetzung nach Anspruch 12, dadurch gekennzeichnet, daß in der Formel (VIII) R₁₆ eine geradkettige oder verzweigte Alkylgruppe und/oder Alkenylgruppe mit 9 bis 14 Kohlenstoffatomen bedeutet, t den Wert Null hat, G Glucose bedeutet und v einen Wert von 1 bis 4 aufweist.

14. Zusammmensetzung nach einem der vorhergehenden Ansprüche, dadurch gekennzeichnet, daß das amphotere oder zwitterionische Tensid aus der Gruppe der Betaine und insbesondere aus den (C₈₋₂₀)-Alkylbetainen, den Sulfobetainen, den (C₈₋₂₀)-Alkyl-(C₁₋₆)-amidoalkylbetainen oder den (C₈₋₂₀)-Alkyl-(C₁₋₆)-amidoalkylsulfobetainen ausgewählt ist.

15. Zusammmensetzung nach einem der vorhergehenden Ansprüche, dadurch gekennzeichnet, daß das vernetzte Methacryloyloxyethyltrimethylammoniumchlorid-Polymer ein vernetztes Copolymer von Methacryloyloxyethyltrimethylammoniumchlorid und Acrylamid ist.

16. Zusammmensetzung nach einem der vorhergehenden Ansprüche, dadurch gekennzeichnet, daß der C₁₄₋₂₂-Fettalkohol in Gewichtsanteilen im Bereich von 1 bis 10 Gew.-% vorliegt, bezogen auf das Gesamtgewicht der Zusammensetzung.

17. Zusammmensetzung nach einem der vorhergehenden Ansprüche, dadurch gekennzeichnet, daß das kationische Tensid vom Typ eines quaternären Ammoniumsalzes in Gewichtsanteilen im Bereich von 0,3 bis 10 Gew.-% vorliegt, bezogen auf das Gesamtgewicht der Zusammensetzung.

18. Zusammmensetzung nach einem der vorhergehenden Ansprüche, dadurch gekennzeichnet, daß das kationische Silicon in Gewichtsanteilen im Bereich von Null bis 10 Gew.-% vorliegt, bezogen auf das Gesamtgewicht der Zusammensetzung.

19. Zusammmensetzung nach einem der vorhergehenden Ansprüche, dadurch gekennzeichnet, daß das nichtionische Tensid vom Alkylpolyglykosidtyp in Gewichtsanteilen im Bereich von 5 bis 30 Gew.-% vorliegt, bezogen auf das Gesamtgewicht der Zusammensetzung.

20. Zusammmensetzung nach einem der vorhergehenden Ansprüche, dadurch gekennzeichnet, daß das amphotere oder zwitterionische Tensid in Gewichtsanteilen im Bereich von Null bis 10 Gew.-% vorliegt, bezogen auf das Gesamtgewicht der Zusammensetzung.

21. Zusammmensetzung nach einem der vorhergehenden Ansprüche, dadurch gekennzeichnet, daß das Glykolmonostearat oder das Glykoldistearat in Gewichtsanteilen im Bereich von 0,1 bis 6 Gew.-% vorliegt, bezogen auf das Gesamtgewicht der Zusammensetzung.

22. Zusammmensetzung nach einem der vorhergehenden Ansprüche, dadurch gekennzeichnet, daß das vernetzte Methacryloyloxyethyltrimethylammoniumchlorid-Polymer in Gewichtsanteilen im Bereich von 0,2 bis 3 Gew.-% vorliegt, bezogen auf das Gesamtgewicht der Zusammensetzung.

23. Zusammmensetzung nach einem der vorhergehenden Ansprüche, dadurch gekennzeichnet, daß sie in Form einer mehr oder weniger verdickten Flüssigkeit, einer Creme oder eines Gels vorliegt.

24. Verwendung der wie in einem der vorhergehenden Ansprüche definierten Zusammensetzung zur gleichzeitigen Pflege und Wäsche der Haare.
